# EUROPEAN PATENT APPLICATION

(11) **EP 2 065 818 A1**
(43) Date of publication of application: **03.06.2009**
(21) Application number: 07807609.8
(22) Date of filing: 20.09.2007
(51) Int. Cl.: G06F 17/50, C40B 30/02, G01N 33/48

(54) **METHOD OF SEARCHING FOR LIGAND**

(30) Priority: 21.09.2006 JP 2006255759
(71) Applicant: Astellas Pharma Inc., Tokyo 103-8411 (JP)
(72) Inventor: FUJITA, Shigeo, Tokyo 103-8411 (JP); ORITA, Masaya, Tokyo 103-8411 (JP)
(74) Representative: Bates, Philip Ian
(86) International application number: PCT/JP2007/068245
(87) International publication number: WO 2008/035729

(57) **Abstract**

Disclosed is a method of searching for a ligand capable of binding to a target biomacromolecule, comprising the step of:
(1) subjecting a number of low-molecular compounds to docking simulation, based on three-dimensional structural data concerning the low-molecular compounds and three-dimensional structural data concerning a ligand-binding region of the target biomacromolecule, to calculate a docking score for each of the low-molecular compounds, and simultaneously acquire three-dimensional positional data which enable each of the low-molecular compounds to stably bind within the ligand-binding region,
(2) acquiring, from among the three-dimensional positional data obtained, all three-dimensional positional data concerning one or more molecular fragments, with respect to each of low-molecular compounds belonging to a higher group based on docking scores,
(3) counting the three-dimensional positional data concerning each molecular fragment obtained, for each of the molecular fragments,
(4) selecting the type and the three-dimensional positional data of a molecular fragment which shows a localization tendency, based on the counting data, and
(5) selecting one or more molecular fragments from among characteristic molecular fragments determined, and determining a compound which satisfies the characteristic molecular fragments.

## Description

### TECHNICAL FIELD

The present invention relates to a method of searching for a ligand capable of binding to a target biomacromolecule. Further, the present invention relates to a method of determining molecular fragments characteristic of a ligand capable of binding to a target biomacromolecule, and three-dimensional positional data of the molecular fragment, as preliminary steps in the above-mentioned ligand search method.

### BACKGROUND ART

As a method of searching for ligands capable of binding a target bioamacromolecule using a computer, a computational method is known, in which, based on three-dimensional data concerning a ligand-binding site located in the target biomacromolecule and a ligand, a valid binding state of the ligand and its docking score are calculated using a docking method, and this calculation is sequentially repeated for each of a number of ligands, to find a ligand having a possibility to bind or to screen out a compound not having a possibility to bind. This method is called a virtual screening or an in silico screening.

Some docking programs are commercially available, and docking scores are calculated in accordance with each specific scoring function used in the docking programs (typical programs include DOCK, GOLD, FlexX, AutoDOCK, DrugScore, and the like). A prediction of a binding state of a ligand in a ligand-binding site of a target biomacromolecule utilizing the docking scores is fairly good. For example, non-patent reference 1 discloses that the results of X-ray crystallographic analysis were reproduced in approximately 80% of examples to be examined. However, non-patent reference 1 also discloses that the binding affinity of a compound could be predicted using the docking scores in some cases, but in most cases good prediction could not be obtained.

Further, there is a method, called a pharmacophore modeling search, in which three-dimensional data concerning a target biomacromolecule are not utilized, but a ligand capable of binding to a target biomacromolecule is searched for using a computer based on three-dimensional data of one or more known ligands. In this method, based on a known active ligand molecule or a superposition of two or more known active ligands, functional groups necessary to interact with a target biomacromolecule (for example, hydrogen-bonding substituent groups, ionic atoms, hydrophobic substituent groups, or the like), and their relative configurations are predicted, and a novel ligand which satisfies the predictions are searched for using a computer (typical programs include Catalyst, Unity, MOE, and the like). However, the use of this pharmacophore modeling search is limited, because calculation is impossible unless one or more known active ligands exist. In addition, the pharmacophore modeling search can provide a novel ligand having properties similar to those of known ligands, but does not directly treat data concerning the interaction between ligands and a target biomacromolecule.

[non-patent reference 1] Journal of Medicinal Chemistry, U.S.A., 2004, vol.47, no.12, p.3032-47

### DISCLOSURE OF INVENTION

### PROBLEMS TO BE SOLVED BY THE INVENTION

As described above, the conventional pharmacophore modeling search needs data concerning one or more known active ligands.
Further, the correlations between docking scores and binding affinities were low in the virtual screening or in silico screening. Although it is preferable that drug candidates useful as a lead compound for drug discovery exhibit IC₅₀ of less than 1 µmol/L, it is not easy to find drug candidates having such a high activity using the conventional virtual screening or in silico screening, or a high throughput screening.
An object of the present invention is to provide a method which enables one, or two or more drug candidates having a high activity for a target biomacromolecule to be provided at a high probability of accuracy.

### MEANS FOR SOLVING THE PROBLEMS

The present invention relates to
[1] a method of determining a molecular fragment characteristic of a ligand capable of binding to a target biomacromolecule, and three-dimensional positional data of the molecular fragment, comprising the steps of:
   (a) subjecting a number of low-molecular compounds to docking simulation, based on three-dimensional structural data concerning the low-molecular compounds and three-dimensional structural data concerning a ligand-binding region of the target biomacromolecule, to calculate a docking score for each of the low-molecular compounds, and simultaneously acquire three-dimensional positional data which enable each of the low-molecular compounds to stably bind within the ligand-binding region (hereinafter referred to as docking simulation step),
   (b) acquiring, from among the three-dimensional positional data within the ligand-binding region obtained in step (a), all three-dimensional positional data concerning one or more predetermined molecular fragments, with respect to each of low-molecular compounds belonging to a higher group based on docking scores calculated in step (a) (hereinafter referred to as acquisition step),
   (c) counting the three-dimensional positional data concerning each molecular fragment obtained in step (b), for each of the molecular fragments (hereinafter referred to as counting step), and
   (d) selecting the type and the three-dimensional positional data of a molecular fragment which shows a localization tendency within the ligand-binding region, based on the counting data obtained in step (c) (hereinafter referred to as selection step);
[2] a method of searching for a ligand capable of binding to a target biomacromolecule, comprising the step of:
   selecting one or more molecular fragments from among characteristic molecular fragments determined by the method of [1], and determining a compound which satisfies the characteristic molecular fragments (hereinafter referred to as ligand determination step);
[3] a program for determining a molecular fragment characteristic of a ligand capable of binding to a target biomacromolecule, and three-dimensional positional data of the molecular fragment, said program making a computer execute the procedures of:
   generating docking atomic coordinates for determining a localized molecular fragment, for each of a number of low-molecular compounds,
   acquiring all three-dimensional positional data concerning one or more predetermined molecular fragments, from among the docking atomic coordinates,
   counting the obtained three-dimensional positional data concerning each molecular fragment, for each of the molecular fragments, and
   selecting the type and the three-dimensional positional data of a molecular fragment which shows a localization tendency, based on the counting data;
[4] a program for searching for a ligand capable of binding to a target biomacromolecule, said program making a computer execute the procedures of:
   generating docking atomic coordinates for determining a localized molecular fragment, for each of a number of low-molecular compounds,
   acquiring all three-dimensional positional data concerning one or more predetermined molecular fragments, from among the docking atomic coordinates,
   counting the obtained three-dimensional positional data concerning each molecular fragment, for each of the molecular fragments,
   selecting the type and the three-dimensional positional data of a molecular fragment which shows a localization tendency, based on the counting data,
   generating docking atomic coordinates of each of one or more physiologically active candidates,
   comparing the docking atomic coordinates of each physiologically active candidate with the type and the three-dimensional positional data of each localized molecular fragment selected, to estimate a localized molecular fragment sufficiency level for the physiologically active candidate, and
   select a physiologically active candidate having a high localized molecular fragment sufficiency level; and
[5] a program for searching for a ligand capable of binding to a target biomacromolecule, said program making a computer execute the procedures of:
   generating docking atomic coordinates for determining a localized molecular fragment, for each of a number of low-molecular compounds,
   acquiring all three-dimensional positional data concerning one or more predetermined molecular fragments, from among the docking atomic coordinates,
   counting the obtained three-dimensional positional data concerning each molecular fragment, for each of the molecular fragments,
   selecting the type and the three-dimensional positional data of a molecular fragment which shows a localization tendency, based on the counting data,
   generating three-dimensional atomic coordinates of each of one or more physiologically active candidates,
   comparing the three-dimensional atomic coordinates of each physiologically active candidate with the type and the three-dimensional positional data of each localized molecular fragment selected, to estimate a localized molecular fragment sufficiency level for the physiologically active candidate, and
   select a physiologically active candidate having a high localized molecular fragment sufficiency level.

The present invention is a method characterized in:
(1) that data concerning known active ligands necessary for the pharmacophore modeling search is unnecessary,
(2) that the interaction with a target biomacromolecule is treated utilizing docking, but docking scores with questionable reliability are not finally used, and
(3) that a ligand is searched for based on a novel concept of molecular fragments characteristic of a ligand capable of binding to a target biomacromolecule, and three-dimensional positional data of the molecular fragment.

### EFFECTS OF THE INVENTION

According to the present invention, one, or two or more drug candidates having a high activity for a target biomacromolecule can be found at a high probability.

### BRIEF DESCRIPTION OF THE DRAWINGS

Figure 1 schematically illustrates the localized positions of benzene rings within the ligand (TIBO)-binding region of HIV-1 reverse transcriptase, determined by the method of the present invention using a DOCK program, together with the binding state of TIBO.
Figure 2 schematically illustrates the localized positions of methyl groups within the ligand (TIBO)-binding region of HIV-1 reverse transcriptase, determined by the method of the present invention using a DOCK program, together with the binding state of TIBO.
Figure 3 schematically illustrates the localized positions of thiocarbonyl groups within the ligand (TIBO)-binding region of HIV-1 reverse transcriptase, determined by the method of the present invention using a DOCK program, together with the binding state of TIBO.
Figure 4 schematically illustrates the localized positions of benzene rings within the ligand (TIBO)-binding region of HIV-1 reverse transcriptase, determined by the method of the present invention using a GOLD program, together with the binding state of TIBO.
Figure 5 schematically illustrates the localized positions of methyl groups within the ligand (TIBO)-binding region of HIV-1 reverse transcriptase, determined by the method of the present invention using a GOLD program, together with the binding state of TIBO.
Figure 6 schematically illustrates the localized positions of thiocarbonyl groups within the ligand (TIBO)-binding region of HIV-1 reverse transcriptase, determined by the method of the present invention using a GOLD program, together with the binding state of TIBO.
Figure 7 schematically illustrates the binding state of a compound (MayBridge, code no. JFD 01710) within the ligand-binding region of HIV-1 reverse transcriptase, the compound being selected by the method of the present invention based on the localized positions (according to the DOCK program) of benzene rings shown in Figure 1.
Figure 8 schematically illustrates the binding state of a compound (MayBridge, code no. JFD 01710) within the ligand-binding region of HIV-1 reverse transcriptase, the compound being selected by the method of the present invention based on the localized positions (according to the GOLD program) of benzene rings shown in Figure 1.
Figure 9 illustrates the structural formulae and the activities (IC₅₀ values) of drug candidates for HIV-1 reverse transcriptase, determined by the method of the present invention.
Figure 10 schematically illustrates the localized positions of benzene rings within the ligand-binding region of CysLT2 receptor, determined by the method of the present invention using a DOCK program, together with the binding state of a compound (Specs, code no. AK-968/40708060) selected by the method of the present invention.
Figure 11 schematically illustrates the localized positions of benzene rings within the ligand-binding region of CysLT2 receptor, determined by the method of the present invention using a DOCK program, together with the binding state of a compound (Specs, code no. AK-968/40708060) selected by the method of the present invention.
Figure 12 illustrates the structural formulae and the activities (IC₅₀ values) of drug candidates for CysLT2 receptor, determined by the method of the present invention.
Figure 13 is a flow chart showing a procedure of the method of the present invention.

### BEST MODE FOR CARRYING OUT THE INVENTION

The present invention includes a method of determining one or more molecular fragments characteristic of a ligand capable of binding to a target biomacromolecule, and three-dimensional positional data of the molecular fragment (hereinafter collectively referred to as characteristic molecular fragment data), comprising the docking simulation step, the acquisition step, the counting step, and the selection step, and a method of searching for a ligand capable of binding to a target biomacromolecule, comprising the docking simulation step, the acquisition step, the counting step, the selection step, and the ligand determination step. In the ligand search method, the ligand determination step may be performed, based on one or more molecular fragments and the three-dimensional positional data thereof obtained by the method of determining characteristic molecular fragment data, to determine a ligand capable of binding to a target biomacromolecule.

In the method of the present invention, prior to the docking simulation step, three-dimensional structural data concerning a ligand-binding region of a target biomacromolecule, and three-dimensional structural data concerning multiple (generally 10 or more, preferably 1000 or more) low-molecular compounds used in the docking simulation are provided.

A target biomacromolecule which may be subjected to the method of the present invention is not particularly limited, so long as the biomacromolecule may be utilized as a target for medicaments. Examples of the target biomacromolecule include naturally-occurring proteins (including glycoproteins), nucleic acids, polysaccharides, and derivatives thereof (such as modified proteins).

As the three-dimensional structural data concerning a ligand-binding region of a target biomacromolecule, three-dimensional structural data about the ligand-binding region alone, or three-dimensional structural data about the whole or part (including the ligand-binding region) of the target biomacromolecule may be used, so long as the ligand-binding region of the target biomacromolecule is included. These three-dimensional structural data are not particularly limited, so long as data necessary for performing the docking simulation are contained. As such data, data with respect to atoms which constitute the ligand-binding region, for example, a type, a state, and/or three-dimensional positional data of each of the atoms, may be used. Further, the three-dimensional structural data may be, for example, already available known data, modified data thereof, or newly determined novel data.

More particularly, in the case that the crystal structure of a target biomacromolecule has been determined, three-dimensional data concerning the crystal structure is available from a databank, such as the Protein Data Bank (http://www.rcsb.org/pdb/). The obtained three-dimensional data may be used as three-dimensional structural data of the ligand-binding region, without being processed, but it is generally preferable to appropriately process the obtained data in accordance with a program used in the docking simulation.

For example, because crystal structures determined by X-ray structure analysis contain no hydrogen atoms, it is preferable to add three-dimensional data concerning hydrogen atoms thereto. Addition of hydrogen atoms may be carried out using, for example, a computer-assisted molecular modeling system [Sybyl (product name)(version 6.4); manufactured by Tripos (U.S.A.)]. In the case of crystal structures determined by performing X-ray structure analysis in a state of a complex of a biomacromolecule with its ligand, it is necessary to remove the three-dimensional data concerning the ligand therefrom. Further, addition of atomic charge data with respect to each atom is preferable, and such data can be added, for example, based on force field parameters of AMBER (Assisted Model Building with Energy Refinement) [The Amber biomolecular simulation programs. J Comput Chem. 2005; 26(16):1668-88, and Force fields for protein simulations. Adv Protein Chem. 2003; 66:27-85].

In the case that the crystal structure of a target biomacromolecule has not been determined, but the crystal structure of another biomacromolecule which is predicted to have a three-dimensional structure similar to that of the target biomacromolecule has been already determined, a modeled crystal structure of the target biomacromolecule can be obtained by homology modeling based on the known crystal structure. For example, homology modeling in which the crystal structure of a similar biomacromolecule is used as a template is performed using a computer-assisted molecular modeling system [for example, MOE (product name)(version 2002.03); manufactured by Chemical Computing Group (Canada)] to obtain a modeled crystal structure of the target biomacromolecule. The obtained calculated crystal structure may be processed as previously described, such as addition of hydrogen atoms, removal of a ligand, or addition of charges, if desired.

The term "low-molecular compound" as used herein means a compound having a molecular weight lower than that of a target biomacromolecule, preferably a compound which may be used as one of docking partners (i.e., a ligand) in a docking simulation program as described below, for example, a compound in which the whole of the molecule or at least a part of the molecule can exist in the ligand-binding region of the target biomacromolecule.

In the method of the present invention, three-dimensional structural data concerning generally 10 or more, preferably 1000 or more, low-molecular compounds are provided. The method for selecting such low-molecular compounds is not particularly limited. For example, a threshold level (upper limit) of molecular weights of the low-molecular compounds is predetermined in accordance with the shape and size of a ligand-binding region of a target biomacromolecule, and compounds having a molecular weight lower than the threshold level can be selected as the low-molecular compounds. In this regard, it is not necessary in the present invention to consider whether or not the selected low-molecular compounds are ligands for the target biomacromolecule, in advance. That is, any low-molecular compound may be used in the method of the present invention, regardless of whether or not it is a ligand for the biomacromolecule, and therefore, a target biomacromolecule for which a ligand is unknown may be subjected to the method of the present invention.

Three-dimensional structural data to be provided are not particularly limited, so long as data necessary for performing the docking simulation are contained. As such data, data with respect to atoms which constitute the low-molecular compounds, for example, a type, a state, and/or three-dimensional positional data of each of the atoms, may be used. Further, the three-dimensional structural data may be, for example, already available known data, modified data thereof, newly determined novel data, or a combination thereof.

More particularly, for example, two-dimensional structural formula data may be obtained from various databases, or catalogues of commercially available compounds, and may be converted to three-dimensional structures using a program for generating three-dimensional structures [for example, Concord (product name)(version 4.0.2); manufactured by Tripos (U.S.A.)] to obtain three-dimensional structural data of each low-molecular compound. If desired, three-dimensional structural data in which the conformation is randomized can be obtained by performing an energy minimization calculation, such as random rotation of rotatable single bonds.

In the docking simulation step of the method according to the present invention, a number of the low-molecular compounds whose three-dimensional structural data are provided are subjected to docking simulation, based on the three-dimensional structural data concerning the low-molecular compounds and the three-dimensional structural data concerning a ligand-binding region of the target biomacromolecule, to calculate a docking score for each of the low-molecular compounds, and simultaneously acquire three-dimensional positional data which enable each of the low-molecular compounds to stably bind within the ligand-binding region.

Various programs for docking simulation are known. Examples of such programs which may be used in this step include, for example, docking simulation programs in which input of three-dimensional structural data concerning a ligand-binding region of a target biomacromolecule and three-dimensional structural data concerning a low-molecular compound can provide output of a docking score of the low-molecular compound and three-dimensional positional data of the low-molecular compound in the ligand-binding region (more particularly, three-dimensional positional data of each atom which constitutes the low-molecular compound).

Examples of the docking simulation programs include, for example, (1) programs utilizing a scoring function based on force-field, (2) programs utilizing an experimental scoring function, and (3) programs utilizing a knowledge-based scoring function [Assessing Scoring Functions for Protein-Ligand Interactions. J. Med. Chem. 2004; 47(12):3032-47].

Programs (1) utilize a classical molecular mechanics energy function, and the sum of van der Waals and electrostatic interactions. As examples thereof, CHARMm (Momany, F. A.; Rone, R. Validation of the general-purpose QUANTA. 3.2/CHARMm force-field. J. Comput. Chem. 1992, 13, 888-900), and chemical score in DOCK (Ewing, T. J. A.; Kuntz, I. D. Critical evaluation of search algorithms for automated molecular docking and database screening. J. Comput. Chem. 1997, 18, 1175-1189) are known.

In program (2), hydrogen bonds, ionic interactions, lipophilic interactions, and the like are parameterized, and a weighted sum is calculated. As examples thereof, ChemScore (Eldridge, M. D.; Murray, C. W.; Auton, T.R.; Paolini, G. V.; Mee, R. P. Empirical scoring functions. I: The development of a fast empirical scoring function to estimate the binding affinity of ligands in receptor complexes. J. Comput.-Aided Mol. Des. 1997, 11, 425-445), GOLD (Jones, G; Willett, P.; Glen, R. C. Molecular recognition of receptor sites using genetic algorithm with a description of desolvation. J. Mol. Biol. 1995, 245, 43-53, and Jones, G; Willett, P.; Glen, R. C.; Leach, A. R.; Taylor, R. Development and validation of a genetic algorithm for flexible docking. J. Mol. Biol. 1997, 267, 727-748), and AutoDock (Morris, G. M.; Goodsell, D. S.; Halliday, R.S.; Huey, R.; Hart, W. E.; Belew, R. K.; Olson, A. J. Automated docking using a Lamarckian genetic algorithm and an empirical binding free energy function. J. Comput. Chem. 1998, 19, 1639-1662, Goodsell, D.S.; Olson, A.J. Automated docking of substrates to proteins by simulated annealing. Proteins: Struc., Funct., Genet. 1990, 8, 195-202, and Morris G. M.; Goodsell D. S.; Huey R.; Olson A.J. Distributed automated docking of flexible ligands to proteins: parallel applications of AutoDock 2.4., J. Comput.-Aided Mol. Des. 1996 Aug, 10(4), 293-304) are known.

In programs (3), atom pair interactions between a target protein and a ligand are totalized. A potential for each type of interaction is available from known complex crystal structure data registered in the Protein Data Bank (PDB). As examples thereof, DrugScore (Gohlke, H.; Hendlich, M.; Llebe, G. Knowledge-based scoring function to predict protein-ligand interactions. J. Mol. Biol. 2000, 295, 337-356), and PMF(Muegge, I.; Martin, Y. C. A general and fast scoring function for protein-ligand interactions: A simplified potential approach. J. Med. Chem. 1999, 42, 791-804) are known.

According to these docking simulation programs, a docking score for a low-molecular compound (ligand) as a simulation subject can be calculated. Evaluation criteria of the docking score vary according to an approach method used in each program, but the term "docking score" used herein means an index showing the stability of ligand-binding.

Further, in these docking simulation programs, when three-dimensional structural data concerning a ligand-binding region of a target biomacromolecule, and three-dimensional structural data concerning a low-molecular compound are input, not only the above docking score for the low-molecular compound, but also a binding mode (i.e., three-dimensional positional data of the low-molecular compound in the ligand-binding region) in which a stable binding between the low-molecular compound and the ligand-binding region is expected, can be provided. In this regard, the "three-dimensional positional data of the low-molecular compound in the ligand-binding region" is, more particularly, three-dimensional positional data of all atoms which constitute the low-molecular compound.

After a docking simulation of a low-molecular compound is completed, a subsequent docking simulation of another low-molecular compound can be performed. Similarly, with respect to all of desired low-molecular compounds among the low-molecular compounds whose three-dimensional structural data are provided, docking simulations can be performed in turn.

In the acquisition step of the method according to the present invention, with respect to a higher group based on docking scores calculated in the docking simulation step, all three-dimensional positional data concerning one or more predetermined molecular fragments are acquired from the three-dimensional positional data within the ligand-binding region obtained in the docking simulation step. In this step, the higher group can be appropriately selected in accordance with various factors, such as the type of a target biomacromolecule, the number of low-molecular compounds to be subjected to docking simulation, and a tendency of obtained docking scores. The higher group is generally a group of the top 10% of the low-molecule compounds, preferably a group of the top 30% or higher, more preferably a group of the top 50% or higher. More members a higher group contains, more accurate three-dimensional positional data of molecular fragments can be obtained.

The term "molecular fragment" as used herein means an atom or a group of atoms which can constitute a compound (in particular, low-molecular compound). Examples of the term "molecular fragment" include various basic skeletons [for example, acyclic (for example, straight-chain or branched-chain) hydrocarbon skeleton (group), cyclic (for example, monocyclic, fused polycyclic, bridged cyclic, spiro, or ring assemblies) hydrocarbon skeleton, or heterocyclic skeleton], characteristic atomic groups (for example, benzene ring, amine, carbonyl group, amide, urea, thiourea, hydroxyl group, thiol group, halogen atom, carboxyl group, sulfo group, haloformyl group, carbamoyl group, amidino group, cyano group, formyl group, thiocarbonyl group, amino group, imino group, or the like), and combinations thereof.

In the counting step, the three-dimensional positional data obtained in the acquisition step are counted for each of the molecular fragments. In the selection step, based on the data obtained in the counting step, the type and the three-dimensional positional data of a molecular fragment which shows a localization tendency within the ligand-binding region are selected. The counting procedure is not particularly limited, so long as a three-dimensional position showing significant localization can be specified for each molecular fragment, and may be performed, for example, in accordance with the following procedure.

A spatial region to be calculated, i.e., the ligand-binding region of a target biomacromolecule, is divided into multiple areas [for example, 8000 (=20³) areas to 125000 (=50³) areas]. A molecular fragment is selected from molecular fragments whose three-dimensional positional data have been obtained. With respect to this molecular fragment, all three-dimensional positional data which occur in the higher group are counted for each of the divided areas. After the counting, whether or not there is a divided area showing a significant localization tendency is judged, and the three-dimensional positional data concerning the divided area showing a significant localization tendency is recorded as three-dimensional positional data characteristic of the molecular fragment. In this regard, such a divided area showing a localization tendency is not limited to one area, with respect to one molecular fragment, that is, multiple divided areas showing a localization tendency may be sometimes specified, or there may be sometimes a case that no localization tendency is shown. After the above counting, judgment, and recording for the single molecular fragment are completed, the counting, judgment, and recording for a subsequent molecular fragment are carried out in a similar fashion to specify three-dimensional positional data characteristic of the subsequent molecular fragment. These steps for desired molecular fragments may be repeated in turn to determine the type of a molecular fragment(s) characteristic of the ligand-binding region, and three-dimensional positional data of the molecular fragment(s). With respect to the selection of molecular fragments, for example, molecular fragments in which an importance for drug discovery was reported or suggested may be preferentially selected, or desired molecular fragments may be experimentally or randomly selected.

In the ligand determination step of the method according to the present invention, from among characteristic molecular fragments (hereinafter referred to as localized molecular fragments) selected in the selection step, one or more types of molecular fragments (preferably two or more types of molecular fragments different in type and/or three-dimensional positional data) are selected, and a compound which satisfies the type of the molecular fragments and the three-dimensional positional data thereof at the same time is determined. As a method for the determination, a screening of a compound database may be exemplified.

A compound group or a database to be screened is not particularly limited, so long as it contains necessary three-dimensional structural data, and various databases, catalogues of commercially available compounds, or the like may be screened. More particularly, for example, a low-molecular compound database containing three-dimensional structural data, as used in docking simulation, preferably a low-molecular compound database containing three-dimensional positional data within a ligand-binding region, as obtained in docking simulation (most preferably, a database composed of low-molecular compounds classified into a higher group) may be exemplified.

When a molecular fragment in which a relative positional relationship for a ligand-binding region is specified is provided, as a method of searching for compounds which satisfy the conditions, various programs are known. Examples of the programs include (1) a search method utilizing "localized molecular fragment (the type and relative spatial position of molecular fragment)" and "three-dimensional positional data of each low-molecular compound as a result of docking", and (2) a 3D (molecular structure) search method utilizing "localized molecular fragment" alone.

In search method (1), the type and the three-dimensional positional data of each localized molecular fragment selected as the characteristic molecular fragments in the selection step may be compared with three-dimensional positional data concerning each low-molecular compound to be screened, obtained by docking simulation, to preferentially select ones having a high degree of agreement therebetween (hereinafter referred to as localized molecular fragment sufficiency level). As a docking simulation program to obtain the above three-dimensional positional data concerning each low-molecular compound, for example, various programs exemplified in the docking simulation step may be used. In this regard, the three-dimensional positional data concerning each low-molecular compound may be obtained using the same docking simulation program as that used in the docking simulation step, or another docking simulation program. The localized molecular fragment sufficiency level can be arithmetically calculated, or determined by observing visualized computer graphics (CG). The visual observation based on CG is preferable, because a state of total binding to a target biomacromolecule can be judged simultaneously.

In search method (2), a screening based on localized molecular fragments is performed with respect to each low-molecular compound whose three-dimensional structural data are provided. The three-dimensional structural data concerning each low-molecular compound may be obtained, for example, by obtaining two-dimensional structural formula data from various databases, or catalogues of commercially available compounds, and converting the data to three-dimensional structural data using a program for generating three-dimensional structures [for example, Concord (product name)(version 4.0.2); manufactured by Tripos (U.S.A.)].

Examples of known programs which may be used in search method (1) or (2) include UNITY (Tripos), CATALYST (Accelrys), and MOE (CCG).

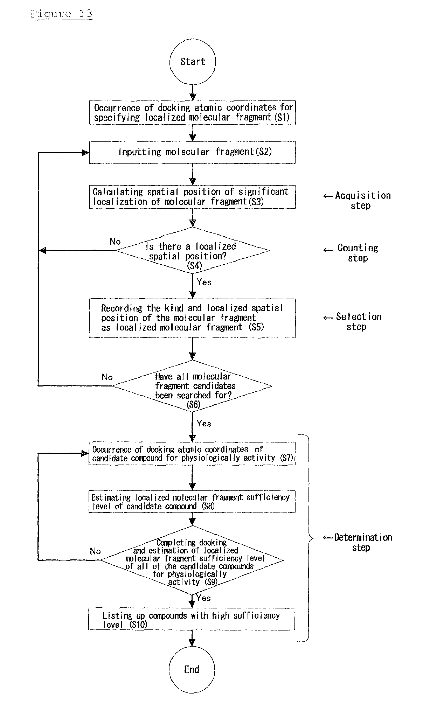

A concrete embodiment of the present invention will be further explained based on the flow chart shown in Figure 13.
Although the flow chart shown in Figure 13 does not include the docking simulation step, each step shown in Figure 13 is carried out after performing the docking simulation step based on three-dimensional structural data of each low-molecular compound and three-dimensional structural data of a ligand-binding region of a target biomacromolecule.

First, low-molecular compounds contained in a higher group are selected based on docking scores obtained in the docking simulation step, and docking atomic coordinates for determining a localized molecular fragment are generated for each of the low-molecular compounds (S1). In this regard, the docking atomic coordinates are data which are converted from the three-dimensional positional data which enable each low-molecular compound to stably bind within the ligand-binding region, obtained in the docking simulation step, to utilize the data in the following steps. The type of a desired molecular fragment is input (S2), and all three-dimensional positional data concerning the molecular fragment are obtained, with respect to each of the docking atomic coordinates for determining a localized molecular fragment, to calculate a significantly localized spatial position of the input molecular fragment (S3). The obtained three-dimensional positional data of the molecular fragment are counted for each divided area within the ligand-binding region, to judge whether or not there is a localized spatial position (S4). In the case that there is no localized spatial position in S4 (i.e., No), return to S2, and input another molecular fragment (S2). Alternatively, in the case that there is a localized spatial position (Yes), continue to S5, and record as a localized molecular fragment the type of the molecular fragment showing a localization, and the localized spatial position of the molecular fragment (S5). Whether or not all desired molecular fragment candidates are searched for is examined (S6). In the case that at least one molecular fragment candidate remains (No), return to S2. In the case that all molecular fragment candidates are completed (Yes), continue to S7.

Each of the physiologically active candidates is subjected to docking simulation to generate the docking atomic coordinates of each candidate (S7). The three-dimensional positional data of each physiologically active candidate is compared with the type and the localized positional data of each localized molecular fragments obtained in S5 to estimate a localized molecular fragment sufficiency level (S8). With respect to all physiologically active candidates, whether or not the docking simulation and the estimation of a localized molecular fragment sufficiency level are completed is examined (S9). In the case that at least a candidate remains in S9 (No), return to S7. In the case that all candidates are completed in S9 (Yes), continue to S10. Based on the obtained localized molecular fragment sufficiency levels, a list of compounds having a high sufficiency level is prepared (S10), and the procedure is completed.

In the flow chart shown in Figure 13, each step (in particular, S7) of the case where search method (1) is used in the ligand determination step is illustrated. In the case that search method (2) is used in the ligand determination step, three-dimensional atomic coordinates of each physiologically active candidate are generated, instead of S7 shown in Figure 13.

### EXAMPLES

To show the effectiveness of the present invention, it was confirmed in the following examples that drug candidates could be searched for based on the three-dimensional structures of target proteins for drug discovery. Concrete procedures will be illustrated in accordance with HIV-1 reverse transcriptase and CysLT2 receptor as embodiments of the present invention, but the present invention is by no means limited to these embodiments.

### Example 1: Searching for drug candidates for HIV-1 reverse transcriptase

### (1) Preparation of data concerning biomacromolecule

In this example, drug candidates for HIV-1 reverse transcriptase were searched.
A crystalline structure of HIV-1 reverse transcriptase was obtained from the Protein Data Bank [http://www.rcsb.org/pdb/, HIV-1 reverse transcriptase (entry 1HNV)]. This crystalline structure was that of a complex with a low-molecular ligand TIBO [5-CHLORO-8-METHYL-7-(3-METHYL-BUT-2-ENYL)-6,7,8,9-TETRAHYDRO-2H-2,7,9A-TRIAZA-BENZO[CD]AZULENE-1-THIONE]. Because this low-molecular ligand binding to HIV-1 reverse transcriptase was unnecessary for the calculation, the three-dimensional structural data concerning TIBO was removed from the original data. Further, the original crystalline structure was determined by X-ray, and thus, did not contain any hydrogen atoms. Hydrogen atoms were added to the data using a computer-assisted molecular modeling system [Sybyl (product name)(version 6.4); manufactured by Tripos (U.S.A.)], and then, all atomic charges were added based on force field parameters of AMBER (Assisted Model Building with Energy Refinement) [The Amber biomolecular simulation programs. J Comput Chem. 2005; 26(16):1668-88, and Force fields for protein simulations. Adv Protein Chem. 2003; 66:27-85] developed by the group of Dr. Coleman at the University of California.

### (2) Preparation of data concerning low-molecular compounds

In searching for drug candidates, commercially available compounds were used as subjects to be calculated. With respect to compounds registered in compound catalogues for high throughput screening [MayBridge (United Kingdom): August, 1999 ed., 50,361 compounds; and Specs (the Netherlands): April, 1999 ed., 71,162 compounds] in sd file format (hereinafter referred to as catalogue compounds), each sd file format data was converted into three-dimensional structural data using a program for generating three-dimensional structures [Concord (product name)(version 4.0.2); manufactured by Tripos (U.S.A.)], and then, an energy minimization calculation was carried out by randomly rotating rotatable single bonds. Drug candidates were searched for from among the three-dimensional structures of catalogue compounds as obtained by the above procedure.

### (3) Docking simulation

The binding site of TIBO located in HIV-1 reverse transcriptase is known as an allosteric site of the enzyme, and some drugs capable of binding to this region are known. With respect to the allosteric site, a docking simulation program developed by the group of Dr. Kuntz at University of California at San Francisco [DOCK (product name)(version 4.0); Ewing, T. J. A.; Kuntz, I.D. Critical evaluation of search algorithms for automated molecular docking and database screening. J. Comput. Chem. 1997, 18, 1175-1189], or a docking simulation program [GOLD (product name)(version 1.0); manufactured by the Cambridge Crystallographic Data Centre (United Kingdom)] was used to carry out docking simulation for each of the catalogue compounds in which three-dimensional structures were generated in step (2). With respect to all catalog compounds, in both simulations using DOCK and GOLD, docking scores concerning each catalogue compound, and three-dimensional positional data of each molecular fragment were obtained. In this regard, DOCK can handle plural scores, and the chemical score of DOCK was used in this Example and the following Example 2.

### (4) Counting molecular fragment data

From among the docking results obtained by each simulation using DOCK and GOLD, about half (approximately 50,000) of compounds having a high docking score were extracted, and the three-dimensional positional data concerning each of typical molecular fragments, such as benzene ring, amine, carbonyl group, amide, urea, thiourea, methyl group, hydroxyl group, thiol group, or the like, were counted for each molecular fragment. As a result, it was found that at least three types of molecular fragments, i.e., benzene ring, methyl group, and thiocarbonyl group, were localized within the pocket of allosteric site of HIV-1 reverse transcriptase. This result was independent of the docking program used, that is, the computational result obtained by the simulation using DOCK was the same as that obtained by the simulation using GOLD.

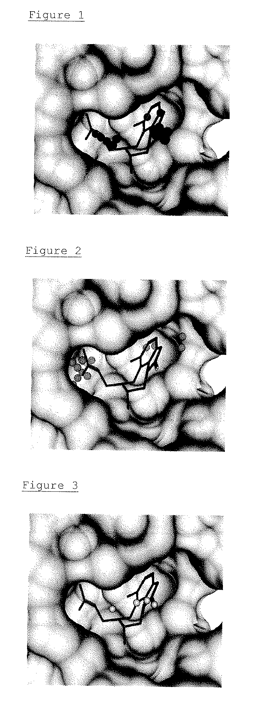

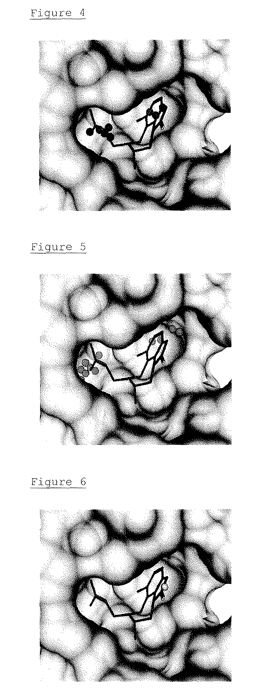

The results are shown in Figures 1 to 6. Figures 1 to 3 show the results in the case that DOCK was used as the docking simulation program. Figures 4 to 6 show the results in the case that GOLD was used as the docking simulation program. Figures 1 and 4 show the results concerning benzene ring, Figures 2 and 5 show the results concerning methyl group, and Figures 3 and 6 show the results concerning thiocarbonyl group. In Figures 1 to 6, the molecular fragments (benzene ring, methyl group, or thiocarbonyl group) are represented by spheres. In addition, Figures 1 to 6 shows TIBO, in which a state of binding was revealed by X-ray crystallographic analysis.
For example, in Figure 1, the spheres (total = 12) shown in Figure 1 indicate the top 12 (twelve) divided areas, among divided areas in which a localization tendency occurred by correcting data for each divided area and examining the level of localization statistically.

As shown in Figures 1 to 6, it is found that the benzene ring, the methyl groups, and the thiocarbonyl group contained in TIBO accord well with the localized areas of benzene ring, methyl group, and thiocarbonyl group determined by the method of the present invention. It is known that these functional groups (molecular fragments) are important for the binding activity of TIBO to the allosteric site of HIV-1 reverse transcriptase, and it was confirmed that compounds which satisfy a molecular fragment(s) determined by the method of the present invention are expected to have a binding activity to a target protein for drug discovery, that is, that the method of the present invention is effective in searching for drug candidates.

### (5) Determination of ligands

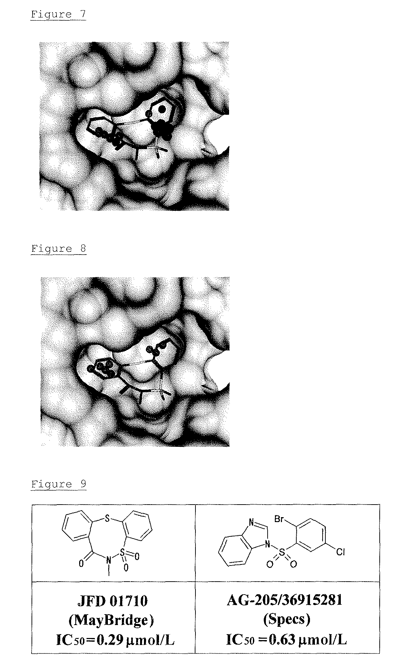

Based on the positional data of the obtained localized molecular fragments, compounds in a binding state which satisfies these conditions were extracted from among three-dimensional coordinates concerning binding states obtained by each simulation using DOCK or GOLD. With respect to a compound (MayBridge, code no. JFD 01710) extracted by using the positional data concerning benzene ring as the molecular fragments, the binding state of the compound is shown in Figures 7 and 8. Figure 7 shows the result extracted by the simulation using DOCK, and Figure 8 shows the result extracted by the simulation using GOLD, and the same result could be extracted. Finally, 36 compounds were selected from the compounds registered in the catalogues of MayBridge and Specs, by using molecular fragments other than a benzene ring.

The selected 36 compounds were actually evaluated, and two compounds having an IC₅₀ of less than 1 µmol/L were found. The obtained drug candidates are shown in Figure 9.

### Example 2: Searching for drug candidates for CysLT2 receptor

### (1) Preparation of data concerning biomacromolecule

In this example, drug candidates for a G protein-coupled receptor (GPCR), CysLT2 receptor, were searched for.
Because X-ray crystallographic analysis of the CysLT2 receptor has not been reported, the three-dimensional structure of the CysLT2 receptor was constructed by homology modeling. From among crystalline structures registered in the Protein Data Bank (http://www.rcsb.org/pdb/), the crystalline structure of bovine rhodopsin (entry 1F88) was obtained. Bovine rhodopsin is the sole GPCR analyzed by X-ray crystallographic analysis, and it is supposed that its crystalline structure is the one which has the most similar three-dimensional structure to that of the CysLT2 receptor belonging to the same GPCRs. The crystalline structure of bovine rhodopsin was that of a complex with retinal. Because this low-molecular ligand binding to rhodopsin was unnecessary for the calculation, the three-dimensional structural data concerning retinal was removed from the original data. Further, because the loop (amino acids of Trp175 to Asn199) between the fourth and the fifth helices capped the pocket of the crystalline structure so as to cover the retinal, and interfered with docking simulation, the amino acids which formed the loop region were also removed from the original data. The resulting three-dimensional structure of bovine rhodopsin was used to a template, and homology modeling was carried out using a computer-assisted molecular modeling system [MOE (product name)(version 2002.03); manufactured by Chemical Computing Group (Canada)] to obtain a three-dimensional structure of the CysLT2 receptor.

Hydrogen atoms were added to the resulting three-dimensional structure using a computer-assisted molecular modeling system [Sybyl (product name)(version 6.4); manufactured by Tripos (U.S.A.)], and then, all atomic charges were added based on force field parameters of AMBER (Assisted Model Building with Energy Refinement) [The Amber biomolecular simulation programs. J Comput Chem. 2005; 26(16):1668-88, and Force fields for protein simulations. Adv Protein Chem. 2003; 66:27-85] developed by the group of Dr. Coleman at the University of California.

### (2) Preparation of data concerning low-molecular compounds

In searching for drug candidates, commercially available compounds were used as subjects to be evaluated. With respect to compounds registered in compound catalogues for high throughput screening [MayBridge (United Kingdom): August, 1999 ed., 50,361 compounds; and Specs (the Netherlands): June, 2003 ed., 174, 245 compounds] in sd file format (hereinafter referred to as catalogue compounds), each sd file format data was converted into three-dimensional structural data using a program for generating three-dimensional structures [Concord (product name)(version 4.0.2); manufactured by Tripos (U.S.A.)], and then, an energy minimization calculation was carried out by randomly rotating rotatable single bonds. Drug candidates were searched for from among the three-dimensional structures of catalogue compounds as obtained by the above procedure.

### (3) Docking simulation

With respect to the binding pocket located in CysLT2 receptor obtained by homology modeling (the region corresponding to that to which retinal binds in the crystalline structure of bovine rhodopsin, a docking simulation program developed by the group of Dr. Kuntz at the University of California at San Francisco [DOCK (product name)(version 4.0); Ewing, T. J. A.; Kuntz, I.D. Critical evaluation of search algorithms for automated molecular docking and database screening. J. Comput. Chem. 1997, 18, 1175-1189], or a docking simulation program [GOLD (product name)(version 1.0); manufactured by the Cambridge Crystallographic Data Centre (United Kingdom)] was used to carry out docking simulation for each of the catalogue compounds in which three-dimensional structures were generated in step (2). With respect to all catalog compounds, in both simulations using DOCK and GOLD, docking scores concerning each catalogue compound, and three-dimensional positional data of each molecular fragment were obtained.

### (4) Counting molecular fragment data

From among the docking results obtained by each simulation using DOCK and GOLD, about 30% (approximately 50,000) of the compounds having a high docking score were extracted, and the three-dimensional positional data concerning each of typical molecular fragments, such as benzene ring, amine, carbonyl group, amide, urea, thiourea, methyl group, hydroxyl group, thiol group, or the like, were counted for each molecular fragment. As a result, it was found that at least the benzene ring was localized within the binding pocket of CysLT2 receptor.

### (5) Determination of ligands

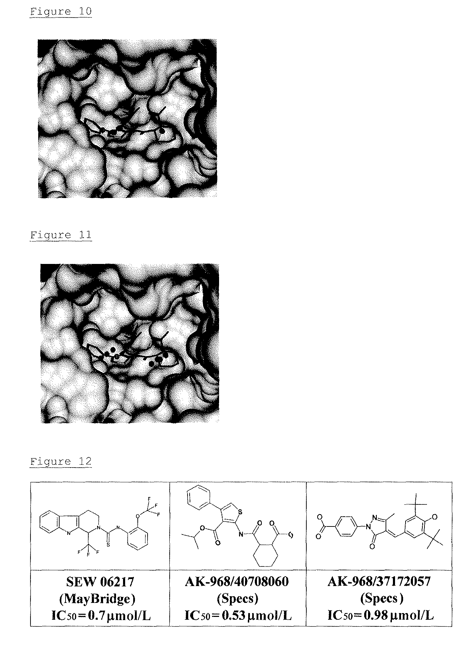

Based on the positional data of the obtained localized molecular fragments, compounds in a binding state which satisfies these conditions were extracted from among three-dimensional coordinates concerning binding states obtained by each simulation using DOCK or GOLD. With respect to a compound (Specs, code no. AK-968/40708060) extracted by using the positional data concerning benzene ring as the molecular fragments, the binding state of the compound is shown in Figures 10 and 11. Figure 10 shows the result extracted by the simulation using DOCK, and Figure 11 shows the result extracted by the simulation using GOLD, and the same result could be extracted with both programs. Finally, 780 compounds were selected from the compounds registered in the catalogues of MayBridge and Specs, by using molecular fragments other than the benzene ring.

The selected 780 compounds were actually evaluated, and three compounds having an IC₅₀ of less than 1 µmol/L were found. The obtained drug candidates are shown in Figure 12.

As described above, it was revealed that useful drug candidates can be searched for by using the method of the present invention. Further, it was revealed that compound can be searched for by the method of the present invention, independently of docking programs used.

### INDUSTRIAL APPLICABILITY

The present invention can be applied to a use in searching for drug candidates.
Although the present invention has been described with reference to specific embodiments, various changes and modifications obvious to those skilled in the art are possible without departing from the scope of the appended claims.

## Claims

1. A method of determining a molecular fragment characteristic of a ligand capable of binding to a target biomacromolecule, and three-dimensional positional data of the molecular fragment, comprising the steps of:
(a) subjecting a number of low-molecular compounds to docking simulation, based on three-dimensional structural data concerning the low-molecular compounds and three-dimensional structural data concerning a ligand-binding region of the target biomacromolecule, to calculate a docking score for each of the low-molecular compounds, and simultaneously acquire three-dimensional positional data which enable each of the low-molecular compounds to stably bind within the ligand-binding region,
(b) acquiring, from among the three-dimensional positional data within the ligand-binding region obtained in step (a), all three-dimensional positional data concerning one or more predetermined molecular fragments, with respect to each of low-molecular compounds belonging to a higher group based on docking scores calculated in step (a),
(c) counting the three-dimensional positional data concerning each molecular fragment obtained in step (b), for each of the molecular fragments, and
(d) selecting the type and the three-dimensional positional data of a molecular fragment which shows a localization tendency within the ligand-binding region, based on the counting data obtained in step (c).

2. A method of searching for a ligand capable of binding to a target biomacromolecule, comprising the step of:
selecting one or more molecular fragments from among characteristic molecular fragments determined by the method according to claim 1, and determining a compound which satisfies the characteristic molecular fragments.

3. A program for determining a molecular fragment characteristic of a ligand capable of binding to a target biomacromolecule, and three-dimensional positional data of the molecular fragment, said program making a computer execute the procedures of:
generating docking atomic coordinates for determining a localized molecular fragment, for each of a number of low-molecular compounds,
acquiring all three-dimensional positional data concerning one or more predetermined molecular fragments, from among the docking atomic coordinates,
counting the obtained three-dimensional positional data concerning each molecular fragment, for each of the molecular fragments, and
selecting the type and the three-dimensional positional data of a molecular fragment which shows a localization tendency, based on the counting data.

4. A program for searching for a ligand capable of binding to a target biomacromolecule, said program making a computer execute the procedures of:
generating docking atomic coordinates for determining a localized molecular fragment, for each of a number of low-molecular compounds,
acquiring all three-dimensional positional data concerning one or more predetermined molecular fragments, from among the docking atomic coordinates,
counting the obtained three-dimensional positional data concerning each molecular fragment, for each of the molecular fragments,
selecting the type and the three-dimensional positional data of a molecular fragment which shows a localization tendency, based on the counting data,
generating docking atomic coordinates of each of one or more physiologically active candidates,
comparing the docking atomic coordinates of each physiologically active candidate with the type and the three-dimensional positional data of each localized molecular fragment selected, to estimate a localized molecular fragment sufficiency level for the physiologically active candidate, and
select a physiologically active candidate having a high localized molecular fragment sufficiency level.

5. A program for searching for a ligand capable of binding to a target biomacromolecule, said program making a computer execute the procedures of:
generating docking atomic coordinates for determining a localized molecular fragment, for each of a number of low-molecular compounds,
acquiring all three-dimensional positional data concerning one or more predetermined molecular fragments, from among the docking atomic coordinates,
counting the obtained three-dimensional positional data concerning each molecular fragment, for each of the molecular fragments,
selecting the type and the three-dimensional positional data of a molecular fragment which shows a localization tendency, based on the counting data,
generating three-dimensional atomic coordinates of each of one or more physiologically active candidates,
comparing the three-dimensional atomic coordinates of each physiologically active candidate with the type and the three-dimensional positional data of each localized molecular fragment selected, to estimate a localized molecular fragment sufficiency level for the physiologically active candidate, and
select a physiologically active candidate having a high localized molecular fragment sufficiency level.
